# NOUVEAU FASCICULE DE BREVET EUROPEEN

(11) **EP 0 557 399 B2**
(45) Date de publication et mention de la décision concernant l'opposition: **15.04.1998**
(45) Mention de la délivrance du brevet: 21.06.1995
(21) Numéro de dépôt: 91920643.3
(22) Date de dépôt: 15.11.1991
(51) Int. Cl.: A61K 7/06, A61K 7/48, A61K 7/08, C11D 1/66

(54) **COMPOSITION LAVANTE ET MOUSSANTE A BASE DE CERTAINES SILICONES INSOLUBLES ET D'UN ALKYLPOLYGLYCOSIDE ET LEUR APPLICATION EN COSMETIQUE ET EN DERMATOLOGIE**
SCHÄUMENDE WASCHZUSAMMENSETZUNG AUF DER BASIS VON BESTIMMTEN UNLÖSLICHEN SILIKONEN UND EINEM ALKYLPOLYGLYKOSID UND IHRE VERWENDUNG IN DER KOSMETIK UND DERMATOLOGIE
FOAMABLE WASHING COMPOSITION BASED ON SELECTED INSOLUBLE SILICONES AND AN ALKYLPOLYGLYCOSIDE, AND COSMETIC AND DERMATOLOGICAL USES THEREOF

(30) Priorité: 15.11.1990 FR 9014223
(43) Date de publication de la demande: 01.09.1993
(73) Titulaire: L'OREAL, 75008 Paris (FR)
(72) Inventeur: DUBIEF, Claude, F-78150 Le Chesnay (FR)
(74) Mandataire: Casalonga, Axel
(86) Numéro de dépôt international: FR9100900
(87) Numéro de publication internationale: WO9208439

(56) Documents cités:
- EP-A- 70 074
- EP-A- 0 194 097
- EP-A- 0 337 354
- EP-A- 0 398 177
- EP-A- 0 418 479
- GB-A- 2 128 627
- Römpp Chemie Lexikon, 8. Aufl., 1987, p. 3856
- Ullmanns Encyklopädie der technischen Chemie, 4. Aufl., Bd. 21, p. 520-521
- Rohm-Haas- technical Bulletin- Triton CG.
- Römpp Chemie Lexikon, 9. Aufl., p. 1737
- "Les silicones, production et application, Rhône-Poulenc Département Silicones, Nouvelle librairie SA, 1988, p. 12-14

## Description

L'invention concerne des compositions lavantes et moussantes à base de certaines silicones insolubles et d'un alkylpolyglycoside et leur application en cosmétique et en dermatologie.

On utilise déjà des huiles siliconées en cosmétique comme lubrifiants dans des compositions de traitement des cheveux ou de la peau ou comme agents "anti-mousses" dans des lotions non rincées. La plupart d'entre elles sont insolubles dans l'eau.

On connaît ègalement des shampooings à base de silicones insolubles dans l'eau et d'agent tensio-actif, les silicones étant dispersées dans le milieu par des alcanolamides d'acides gras, des stéarates d'éthylèneglycol ou des diméthylalkyl(C₁₆-C₂₂)amines N-oxydes.

On connait également des compositions de lavage contenant un dérivé siliconé et un composé alkylsaccharide. Elles sont décrites dans la demande de brevet EP-A-0 398 177.

D'autres compositions contenant un alkylpolysaccharide et un polymère siliconé cationique ont été décrites dans la demande EP-A-0 377 354.

La demanderesse vient de découvrir, d'une manière surprenante, qu'on pouvait obtenir une composition moussante et lavante à base de certaines silicones insolubles dans l'eau, en utilisant, comme agent dispersant et détergent, un alkylpolyglycoside.

L'invention a donc pour objet de nouvelles compositions lavantes et mousssantes à base de silicones insolubles dans l'eau définies ci-après et d'au moins un alkylpolyglycoside cosmétiquement acceptables.

L'invention a également pour objet un procédé de lavage et de conditionnement des cheveux et un procédé de lavage de la peau mettant en oeuvre ces compositions.

D'autres objets apparaîtront à la lecture de la description et des èxemples qui suivent.

La présente invention concerne principalement une composition lavante et moussante, caracérisée par le fait qu'elle comporte dans un milieu aqueux :
a) une silicone insoluble dans ledit milieu et non réactive avec celui-ci, choisie parmi les gommes de silicone, les résines de silicone, les silicones organomodifiées comportant un ou plusieurs groupements thiols, carboxylate, γ-hydroxypropyle, acide alkylcarboxylique, hydroxyalkylsulfonate, hydroxyalkylthiosulfate ou hydroxyacylamino, ainsi que leurs mélanges, à l'exclusion
   (i) des gommes de polydiméthylsiloxane α,ω-triméthyle, et
   (ii) des silicones organo-modifiées répondant à la formule 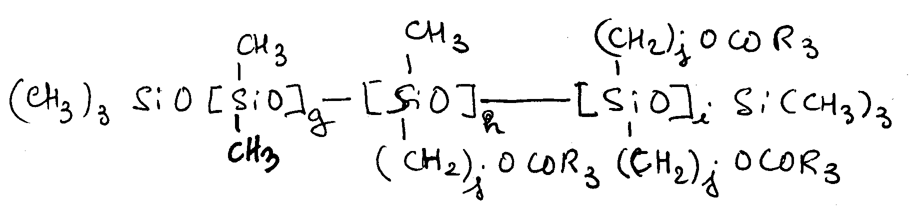 dans laquelle g, h et i sont individuellement un nombre de 1 à 350, j est un nombre de 0 à 10 et R₃ et un groupe alkyle ayant 9 à 21 atomes de carbone et à la formule : 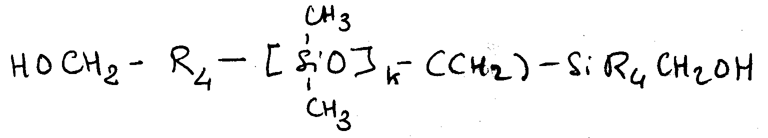 dans laquelle k est un nombre de 1 à 500 et R₄ représente un groupe Cn₄H₂ₙ₄ où n₄ est un nombre de 0 à 4 ;
b) de 4 à 60% d'au moins un composé correspondant à la structure développée (II): 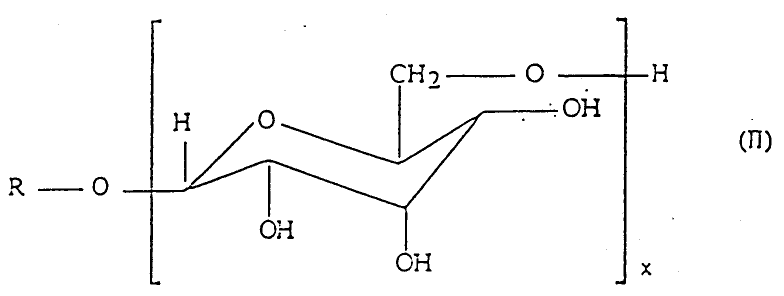 dans laquelle :
   R désigne un radical ou un mélange de radicaux. alkyle ou alkényle à chaîne droite ou ramifiée en C₈-C₂₄;
   x est un nombre de 1 à 15;
      le rapport en poids : composé de formule (II) silicone insoluble étant supérieur ou égal à 1.

Les silicones, utilisées conformément à la présente invention, sont des polyorganosiloxanes insolubles dans les milieux aqueux, pouvant se présenter sous forme d'huiles, de cires, de gommes ou de résines.

Les organopolysiloxanes sont définis plus en détail dans l'ouvrage de Walter NOLL "Chemistry and Technology of Silicones" (1968) Academic-Press.

Ces silicones ne doivent pas être réactives avec lesdits milieux.

Les polysiloxanes utilisés, conformément à l'invention, sont choisis parmi les gommes et résines de silicones, les polysiloxanes organomodifiés définis ci-après ainsi que leurs mélanges.

Les gommes de silicones, conformes à la présente invention, sont des polydiorganosiloxanes de fortes masses moléculaires, comprises entre 200.000 et 1.000.000.

On cite, par exemple, les gommes suivantes :
- poly[(diméthylsiloxane)/(méthylvinylsiloxane)],
- poly[(dimétliylsiloxane)/(diphénylsiloxane)],
- poly[(diméthylsiloxane)/(phénylméthylsiloxane)],
- poly[(diméthylsiloxane)/(diphénylsiloxane)/(méthylvinylsiloxane].

Les résines d'organopolysiloxanes utilisables conformément à l'invention, sont des systèmes siloxaniques réticulés renfermant les unités R'₂SiO_{2/2}, R'SiO_{3/2} et SiO_{4/2}, dans lesquelles R' représente un groupement hydrocarboné possédant 1 à 6 atomes de carbone ou un groupement phényle. Parmi ces produits, ceux particutièrement préférés sont ceux dans lesquels R' désigne un radical alkyle inférieur ou un radical phényle.

Parmi ces résines, on peut citer le produit vendu sous la dénomination DOW CORNING 593 qui est un mélange de triméthylsiloxysilicate et de polydiméthylsiloxane, ou ceux vendus sous les dénominations SILICONE FLUID SS 4230 et SS 4267 par la Société GENERAL ELECTRIC et qui sont des "diméthyl/triméthylpolysiloxane".

Les silicones organomodifiées sont des polyalkylsiloxanes, des polyarylsiloxanes ou des polyalkylarylsiloxanes comportant dans leur structure un ou plusieurs groupements organo-fonctionnels directement fixés sur la chaîne siloxanique ou fixés par l'intermédiaire d'un radical hydrocarboné.

Parmi ces silicones, on peut citer, par exemple, les silicones comportant :
**1-** des groupements thiols comme dans les GP 72 A et GP 71 de GENESEE ou dans le produit SLM 50253/5 de la Société WACKER,
**2-** des groupements carboxylates comme dans les produits décrits dans le brevet EP-A-186.507 de la Société CHISSO CORPORATION,
**3-** des groupements γ-hydroxypropyle, comme les produits décrits dans la demande de brevet français n° FR-85.16.334, répondant à la formule suivante (III) : 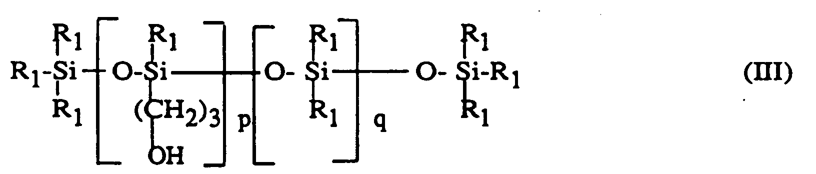 dans laquelle :
   - les radicaux R₁, identiques ou différents, sont choisis parmi les radicaux méthyle et phényle, au moins 60% en mole des radicaux R₁ désignant méthyle;
   - p est compris entre 1 et 30 inclus;
   - q est compris entre 1 et 150 inclus.

   On cite, par exemple, le produit 71615 V 300 vendu par la Société RHONE POULENC.
**4-** des groupements acyloxyalkyle, comme par exemple les polyorganosiloxanes décrits dans la demande de brevet FR-A 2.641.185, répondant à la formule : 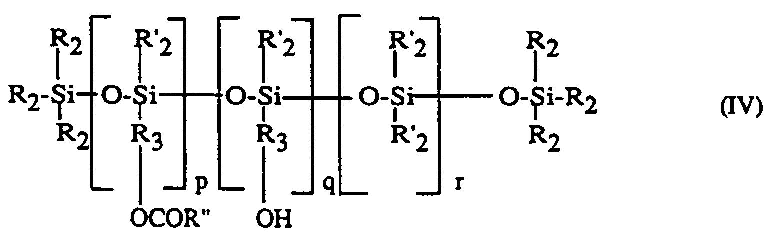 dans laquelle :
   R₂ désigne un groupement méthyle, phényle, -OCOR'', hydroxyle, un seul des radicaux R₂ par atome de silicium peut être OH;
   R'₂ désigne méthyle, phényle, au moins 60% en mole de l'ensemble des radicaux R₂ et R'₂ désignant méthyle;
   R'' désigne un alkyle ou alkényle en C₈-C₂₀;
   R₃ désigne un radical alkylène hydrocarboné divalent, linéaire ou ramifié, en C₂-C₁₈;
   r est compris entre 1 et 120 inclus;
   p est compris entre 1 et 30;
   q est égal à 0 ou est inférieur à 0,5p, p+q étant compris entre 1 et 30; les polyorganosiloxanes de formule (IV) pouvant contenir des groupements 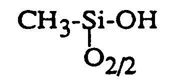 dans des proportions ne dépassant pas 15% de la somme p+q+r.
**5-** des groupements anioniques de type carboxylique tels que les groupements alkylcarboxyliques comme ceux présents dans le produit X-22-3701E de la Société SHIN-ETSU ou dans le produit Silicone Fluid FZ 3703 de la Société UNION CARBIDE; 2-hydroxyalkylsulfonate; 2-hydroxyalkylthiosulfate tels que les produits vendus par la Société GOLDSCHMIDT sous les dénominations "ABIL S201" et "ABIL S255".
**6-** les silicones à fonction hydroxyacylamino choisies notamment parmi les composés de formule : 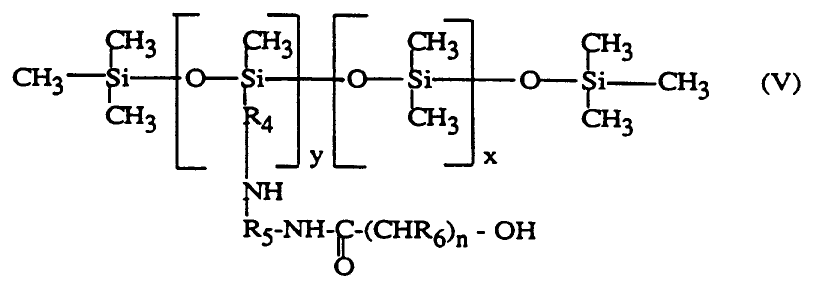 dans laquelle :
   R₄ et R₅ désignent (CH₂)₂, (CH₂)₃, (CH₂)₄, -CH₂CH(CH₃)CH₂- ;
   R₆ désigne hydrogène, hydroxyle, alkyle, hydroxyalkyle;
   n est un nombre entier variant de 2 à 7;
   x est un nombre entier variant de 20 à environ 1500;
   y varie entre environ 0,5 et 10;
      et le rapport y/x est inférieur à 0,05.

De telles silicones sont plus particulièrement décrites dans la demande de brevet européen EP-A-O342834.

Une silicone plus particulièrement préférée est celle répondant à la formule (V), dans laquelle :
R₄ désigne -CH₂CH(CH₃)CH₂- ;
R₅ désigne -(CH₂)₂- ;
R₆ désigne hydrogène;
x est égal à 392;
y est égal à 8;
n est égal à 3.

De telles silicones sont vendues en particulier sous la dénomination Q2-8413 par la Société DOW CORNING.

Les polyorganosiloxanes plus particulièrement préférés, conformément à l'invention, sont les gommes de silicone et les silicones organomodifiées des paragraphes 1, 2 et 5 définis précédemment.

Les silicones insolubles dans l'eau, utilisables conformément à la présente invention et telles que dèfinies ci-dessus, sont présentes dans les compositions dans des proportions comprises entre 0,2 et 30% et de préférence entre 0,4 et 15% par rapport au poids total des compositions.

Les composés alkylpolyglycosides de formule développée (II) définie ci-dessus, utilisés conformément à l'invention, sont de préférence représentés par les produits vendus par la Société HENKEL sous la dénomination APG, tels que les produits APG 300, APG 350, APG 500, APG 550, APG 625, APG base 10-12; les produits vendus par la Société SEPPIC sous les dénominstions TRITON CG 110 (ou ORAMIX CG 110) et TRITON CG 312 (ou ORAMIX NS 10); ceux vendus par la Société BASF sous la dénomination LUTENSOL GD 70.

Les alkylpolyglycosides de formule (II) préférés, selon l'invention, sont l'APG 300 de la Société HENKEL, Le TRITON CG 110 de la Société SEPPIC et le LUTENSOL GD 70 de la Société BASF.

Les compositions cosmétiques, selon l'invention, peuvent être utilisées en particulier comme shampooings, sous forme de gels douche ou de bains moussants pour le corps.

Les compositions cosmétiques conformes à la présente invention, peuvent contenir, en outre, d'autres agents tensio-actifs différents de ceux de formule (II) choisis parmi les agents tensio-actifs anioniques, amphotères, zwittérioniques, non ioniques ou leurs mélanges.

Parmi les agents tensio-actifs anioniques, on peut citer plus particulièrement les sels alcalins, de magnésium, d'ammonium, d'amines ou d'amino alcools des composés suivants :
- les alkylsulfates, les alkyléthersulfates, les alkylamidoéthersulfates, les alkylarylpolyéthersulfates, les monoglycéridesulfates;
- les alkylsulfonates, les alkylamidesulfonates, les alkylarylsulfonates, les oléfines sulfonates, les paraffines sulfonates;
- les alkylsulfosuccinates, les alkyléthersulfosuccinates, les alkylamidesulfosuccinates;
- les alkylsulfosuccinamates;
- les alkylsulfoacétates;
- les alkylphosphates, les alkylétherphosphates;
- les acylsarcosinates, les acyliséthionates, les N-acyltaurates,
   le radical alkyle ou acyle de ces différents composés étant généralement constitué par une chaîne carbonée comportant de 12 à 20 atomes de carbone.

Parmi les agents tensio-actifs anioniques, on peut également citer :
les sels d'acides gras tels que les sels des acides oléique, ricinoléique, palmitique, stéarique; les acides d'huile de coprah ou d'huile de coprah hydrogénée; les acyl lactylates, dont le radical acyle comporte de 8 à 20 atomes de carbone.

Parmi les agents tensio-actifs considérés comme faiblement anioniques, on peut citer :
les acides éthers carboxyliques polyoxyalkylénés, répondant à la formule :

R₇-(OC₃H₆)ₚ-(OC₂H₄)ₙ-OCH₂COOA (VI)

dans laquelle R₇ désigne un radical ou un mélange de radicaux alkyle ou alcényle en C₈-C₂₂, linéaire ou ramifié, un radical alkyl (C₈-C₉)phényle, R'₇CONH-CH₂-CH₂ avec R'₇ désignant un radical alkyle ou alcényle, linéaire ou ramifié, en C₁₁-C₂₁;
n est un nombre entier ou décimal compris entre 2 et 24,
p est un nombre entier ou décimal compris entre 0 et 6,
A désigne un atome d'hydrogène ou bien Na, K, Li, 1/2 Mg ou un reste monoéthanolamine, ammonium ou triéthanolamine.

Les acides éthers carboxyliques polyoxyéthylénés utilisables conformément à l'invention, sont choisis de préférence parmi les composés de formule (VI) dans laquelle R₇ désigne un radical ou un mélange de radicaux alkyl(C₁₀-C₁₈), oléyle, un radical nonylphényle ou octylphényle. A désigne un atome d'hydrogène ou de sodium et p est égal à 0.

On utilise de préférence les produits commerciaux vendus par la Société CHEM Y sous les dénominations AKYPO ou par la Société SANDOZ sous les dénominations SANDOPAN.

Les agents tensio-actifs non-ioniques sont plus particulièrement choisis parmi les alcools, les alkylphénols, les acides gras polyoxy-ethylénés, polypropoxylés ou polyglycérolés, à chaîne grasse comportant 8 à 18 atomes de carbone, le nombre de groupements oxyde d'éthylène ou oxyde de propylène étant compris entre 2 et 50 et le nombre de groupements glycérol étant compris entre 2 et 30.

On peut également citer les copolymères d'oxydes d'éthylène et de propylène, les condensats d'oxydes d'éthylène et de propylène sur des alcools gras, les amides gras polyéthoxylés, ayant de préférence 2 à 30 moles d'oxyde d'éthylène, les amides gras polyglycérolés comportant 1 à 5 groupements glycérol et de préférence 1,5 à 4, les amines grasses polyéthoxylées, ayant de préférence 2 à 30 moles d'oxyde d'éthylène, les esters d'acides gras du sorbitan oxyéthylénés ou non, ayant de préférence 2 à 30 moles d'oxyde d'éthylène, les esters d'acides gras de sucrose, les esters d'acides gras du polyéthylèneglycol, les esters d'acides gras de glycols, les oxydes d'amines tels que les oxydes d'alkylamines ou de N-acylamidopropylmorpholine.

Les agents tensio-actifs amphotères ou zwittérioniques plus particulièrement préférés sont :
- les dérivés d'amines secondaires ou tertiaires aliphatiques, dans lesquels le radical aliphatique est une chaîne linéaire ou ramifiée comportant 8 à 18 atomes de carbone et qui contient au moins un groupe anionique hydrosolubilisant carboxylique, sulfonate, sulfate, phosphate ou phosphonate;
- les alkyl(C₈-C₂₀)bétaïnes, les sulfobétaïnes; les alkyl(C₈-C₂₀) amido alkyl(C₁-C₆)bétaïnes ou les alkyl(C₈-C₂₀)amido alkyl(C₁-C₆) sulfobétaïnes.

Parmi les dérivés d'amines, on peut citer les produits vendus sous la dénomination "MIRANOL", tels que décrits dans les brevets US-A-2.528.378 et 2.781.354 et classés dans le dictionnaire CTFA, 3ème édition, 1982, sous la dénomination d'Amphocarboxyglycinates et Amphocarboxypropionates.

Les bétaïnes sont choisies de préférence parmi les alkyl (C₁₀-C₂₀)bétaïnes.

La quantité en tensio-actifs, hors alkylpolygycoside, dans ces compositions de lavage, est généralement inférieure à 20% en poids par rapport au poids total de la composition.

Les compositions, selon l'invention, présentent un pH généralement compris entre 2 et 9 et plus particulièrement entre 3 et 8.

Les compositions, selon l'invention, peuvent contenir également des agents régulateurs de viscosité, tels que des électrolytes comme le chlorure de sodium ou le xylènesulfonate de sodium, des hydrotropes, des épaississants comme les dérivés de la cellulose, tels que par exemple la carboxyméthylcellulose, l'hydroxypropylcellulose, l'hydroxyéthylcellulose, la gomme de guar, des gommes de guar hydroxypropylées, les scléroglucanes, la gommé de xanthane.

Ces agents régulateurs de viscosité sont utilisés dans des proportions allant jusqu'à 15% en poids par rapport au poids total de la composition et de préférence jusqu'à 6%.

Les compositions conformes a l'invention peuvent éventuellement contenir en outre d'autres agents ayant pour effet d'améliorer les propriétés cosmétiques des cheveux et/ou de la peau, pourvu qu'ils n'altèrent pas la stabilité des compositions, tels que des tensio-actifs cationiques, des polymères, des protéines quaternisées ou non ou des silicones hydrosolubles.

Les polymères, les tensio-actifs cationiques et les protéines quaternisées ou non, sont utilisés dans les compositions cosmétiques ou dermatologiques, selon l'invention, dans des proportions comprises entre 0,05 et 6% et de préférence entre 0,1 et 3% par rapport au poids total de la composition.

Les silicones hydrosolubles peuvent être utilisées dans les compositions selon l'invention; dans des proportions allant jusqu'à 10% et de préférence entre 0,5 et 6% par rapport au poids total de la composition.

Les compositions selon l'invention peuvent également contenir différents adjuvants habituellement utilisés en cosmétique, tels que des parfums, des conservateurs, des séquestrants, des stabilisateurs de mousse, des agents propulseurs, des colorants, des agents acidifiants ou alcalinisants ou d'autres adjuvants selon l'usage envisagé.

Les compositions dermatologiques contiennent en outre une substance active pour le traitement des affections dermatologiques.

Les procédés de lavage et/ou de conditionnement des cheveux ou de la peau consistent à appliquer sur ceux-ci une composition telle que définie ci-dessus, cette application étant suivie d'un rinçage.

Les exemples qui suivent illustrent la présente invention.

### EXEMPLE 1

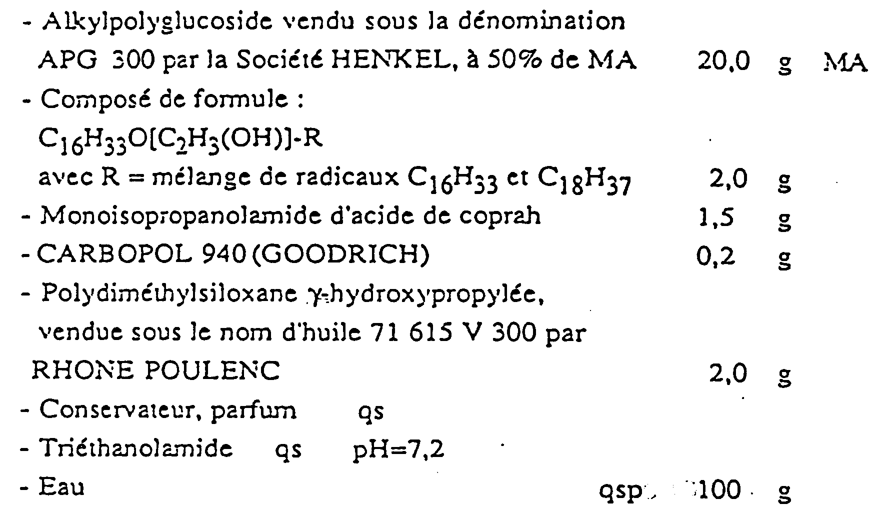

### EXEMPLE 2

| | |
|---|---|
| - APG 300 à 50% de MA (HENKEL) | 12,5 g MA |
| - Distéarate d'éthylèneglycol | 1,5 g |
| - Diéthanolamide d'acide de coprah | 2,0 g |
| - Distéarate de polyéthylèneglycol 6000 | 3,0 g |
| - Polydiméthylsiloxane à groupements mercaptopropyle, vendue sous la dénomination X 28360 par la Société DOW CORNING | 2,5 g |
| - Conservateur, parfum qs | |
| - Acide chlorhydrique qs pH = 7,5 | |
| -Eau qsp | 100 g |

### EXEMPLE 3

| | |
|---|---|
| - APG 300 à 50% de MA (HENKEL) | 15,0 g MA |
| - Composé décrit à l'exemple 1 | 1,5 g |
| - Monoisopropanolamide d'acide de coprah | 2,0 g |
| - CARBOPOL 940 (GOODRICH) | 0,3 g |
| - Polydiméthylsiloxane à groupements amide, vendue sous la dénomination X 28491 par DOW CORNING | 3,0 g |
| - Conservateur, parfum qs | |
| - Acide chlorhydrique qs pH = 7 | |
| - Eau qsp | 100 g |

### EXEMPLE 4

| | |
|---|---|
| - APG 300 à 50% de MA (HENKEL) | 25,0 g MA |
| - Distéarate d'éthylèneglycol | 1,0 g |
| - Monoisopropanolamide d'acide de coprah | 2,0 g |
| - Mélange polydiméthylsiloxane/triméthylsiloxysilicate, vendu sous la dénomination DC 593 par DOW CORNING | 1,0 g |
| - Parfum, conservateur qs | |
| - Acide chlorhydrique qs pH = 7,2 | |
| - Eau qsp | 100 g |

### EXEMPLE 5

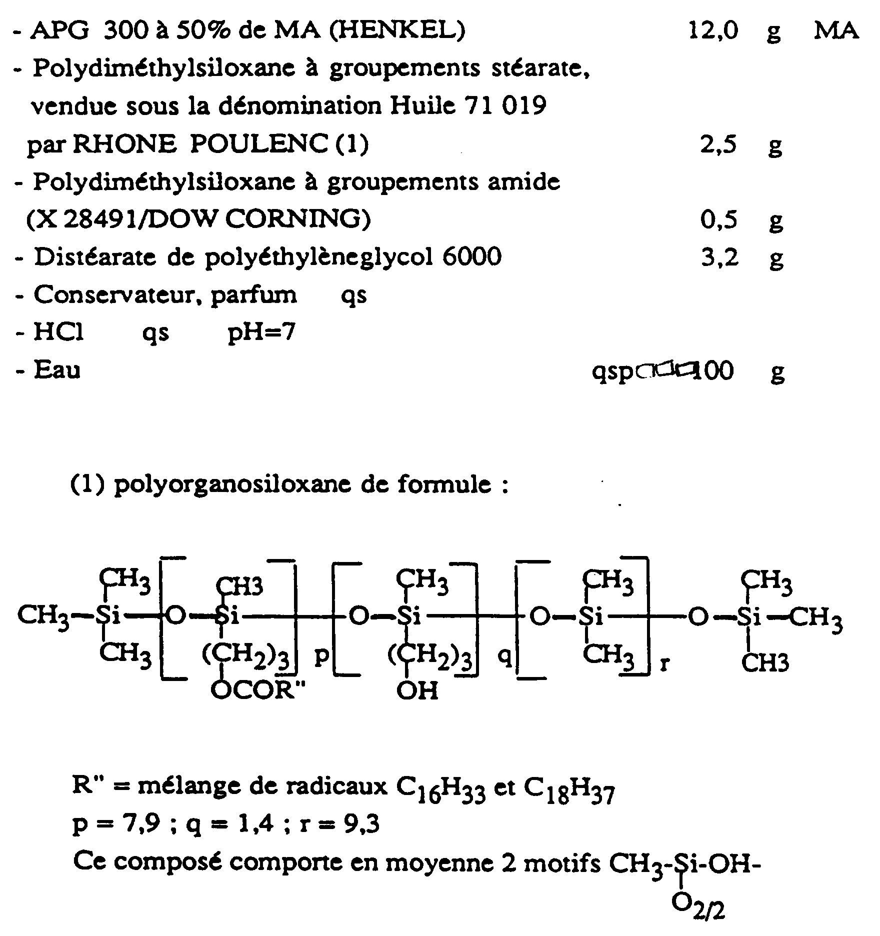

Il peut être préparé comme décrit à l'exemple A de la demande FR 2.641.185.

### EXEMPLE 6

| | |
|---|---|
| - APG 300 à 50% de MA (HENKEL) | 10,0 g MA |
| - Polydiméthylsiloxane à groupements carboxyliques, vendue sous la dénomination FZ 23703 par UNION CARBIBE | 3,0 g |
| - Gomme de xanthane vendue sous la dénomination KELTROL T par KELCO | 1,2 g |
| - Laurylbétaïne à 32% de MA | 5,0 g MA |
| - Parfum, conservateur qs | |
| - pH = 5,7 | |
| - Eau qsp | 100 g |

### EXEMPLE 7

| | |
|---|---|
| - Alkylpolyglycoside vendu sous la dénomination LUTENSOL GD 70 par BASF, à 70% de MA | 30,0 g MA |
| - Polydiméthylsiloxane à fonctions thiols, vendue sous la dénomination SLM 50253/5 par WACKER | 1,0 g |
| - Gomme de xanthane (KELTROL T) | 0,8 g |
| - Alcool laurique polyglycérolé (4 moles) à 60% de MA | 5,0 g MA |
| - Parfum, conservateur qs | |
| - pH = 7,5 | |
| - Eau qsp | 100 g |

### EXEMPLE 8

| | |
|---|---|
| - Alkylpolyglucoside vendu sous la dénomination APG 300 par la Société HENKEL, à 50% de MA | 30,0 g MA |
| - Distéarate d'éthylèneglycol | 2,0 g |
| - Monoisopropanolamide d'acide de coprah | 1,5 g |
| - Gomme de polydiphényldiméthylsiloxane, vendue sous la dénomination de GOMME 761 par la Société RHONE POULENC | 1,0 g |
| - Mélange de tétraméthylhexanes/heptanes/octanes, vendu sous la dénomination d'ISOPAR H par la Société EXXON | 2,0 g |
| - Conservateur, parfum qs | |
| - Eau qsp | 100 g |

Le pH est ajusté à 7,4 par l'acide chlorhydrique.

## Revendications

1. Composition lavante et moussante pour le traitement des cheveux ou de la peau, caractérisée par le fait qu'elle comporte dans un milieu aqueux :
a) une silicone insoluble dans ledit milieu et non réactive avec celui-ci, choisie parmi les gommes de silicone, les résines de silicone, les silicones organomodifiées comportant un ou plusieurs groupements thiols, carboxylate, γ-hydroxypropyle, acide alkylcarboxylique, hydroxyalkylsulfonate, hydroxyalkylthiosulfate ou hydroxyacylamino, ainsi que leurs mélanges à l'exclusion
(i) des gommes de polydiméthylsiloxane α,ω-triméthyle, et
(ii) des silicones organo-modifiées répondant à la formule 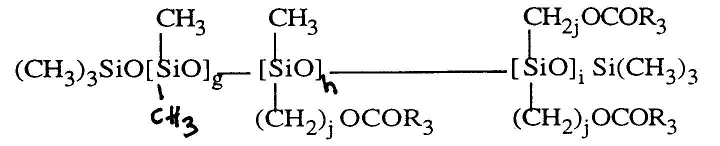 dans laquelle g, h et i sont individuellement un nombre de 1 à 350, j est un nombre de 0 à 10 et R₃ est un groupe alkyle ayant 9 à 21 atomes de carbone et à la formule : 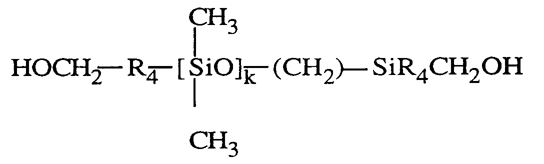 dans laquelle k est un nombre de 1 à 500 et R₄ représente un groupe Cn₄H₂ₙ₄ où n₄ est un nombre de 0 à 4 ;
b) de 4 à 60% d'au moins un composé correspondant à la structure développée suivante : 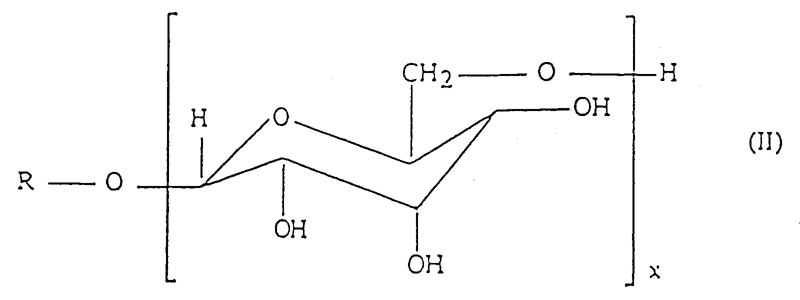 dans laquelle :
R désigne un radical ou un mélange de radicaux alkyle ou alkényle à chaîne droite ou ramifiée en C₈-C₂₄;
x est un nombre de 1 à 15;
le rapport en poids : composé de formule (II)/silicone, étant supérieur ou égal à 1.

2. Composition selon la revendication 1, caractérisée par le fait que la silicone est choisie parmi :
a) les gommes de silicones constituées de polydiorganosiloxanes à poids moléculaire éleyé compris entre 200.000 et 1.000.000; ou
b) les résines d'organopolysiloxanes qui sont des systèmes siloxaniques réticulés, renfermant des unités R'₂SiO_{2/2}, R'SiO_{3/2} et SiO_{4/2}, dans lesquelles R' représente un groupement hydrocarboné possédant 1 à 6 atomes de carbone ou un groupement phényle; ou bien
c) les polyalkylsiloxanes, les polyarylsiloxanes ou les polyalkylarylsiloxanes comportant dans leur structure un ou plusieurs groupements fonctionnels tels que définis dans la revendication 1, fixés sur la chaîne siloxanique ou fixés par l'intermédiaire d'un radical hydrocarboné; ainsi que leurs mélanges.

3. Composition selon la revendication 2, caractérisée par le fait que les gommes de silicone sont choisies parmi les gommes de poly (diméthylsiloxane/méthylvinylsiloxane); de poly(diméthylsiloxane/diphénylsiloxane); de poly(diméthylsiloxane/phénylméthylsiloxane); de poly(diméthylsiloxane/diphénylsiloxane/méthylvinylsiloxane).

4. Composition selon la revendication 1, caractérisée par le fait que les silicones organomodifiées à groupements γ-hydroxypropyle sont des polyorganosiloxanes, répondant à la formule : 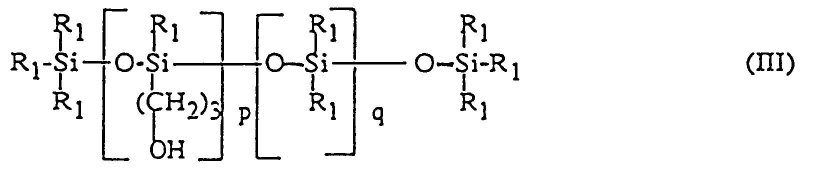 dans laquelle :
- les radicaux R₁, identiques ou différents, sont choisis parmi les radicaux méthyle et phényle, au moins 60% en mole des radicaux R₁ désignant méthyle;
- p est compris entre 1 et 30 inclus;
- q est compris entre 1 et 150 inclus.

5. Composition selon la revendication 1, caractérisée par le fait que les silicones organomodifiées à groupements acyloxyalkyle répondent à la formule : 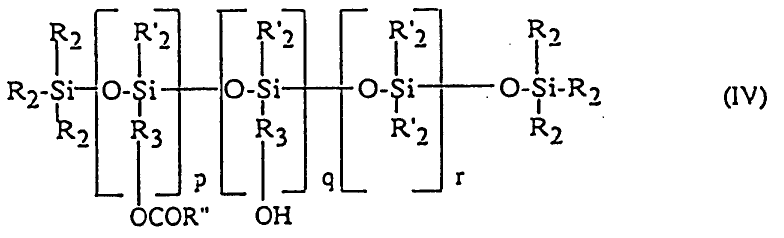 dans laquelle :
R₂ désigne un groupement méthyle, phényle, -OCOR'', hydroxyle, un seul des radicaux R₂ par atome de silicium peut être OH;
R'₂ désigne méthyle, phényle, au moins 60% en mole de l'ensemble des radicaux R₂ et R'₂ désignant méthyle;
R'' désigne un alkyle ou alkényle en C₈-C₂₀;
R₃ désigne un radical alkylène hydrocarboné divalent, linéaire ou ramifié, en C₂-C₁₈;
r est compris entre 1 et 120 inclus;
p est compris entre 1 et 30;
q est égal à 0 ou est inférieur à 0,5p, p+q étant compris entre 1 et 30; les polyorganosiloxanes de formule (IV) pouvant contenir des groupements 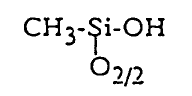 dans des proportions ne dépassant pas 15% de la somme p+q+r.

6. Composition selon la revendication 1, caractérisée par le fait que les silicones organomodifiées à fonction hydroxyacylamino répondent à la formule : 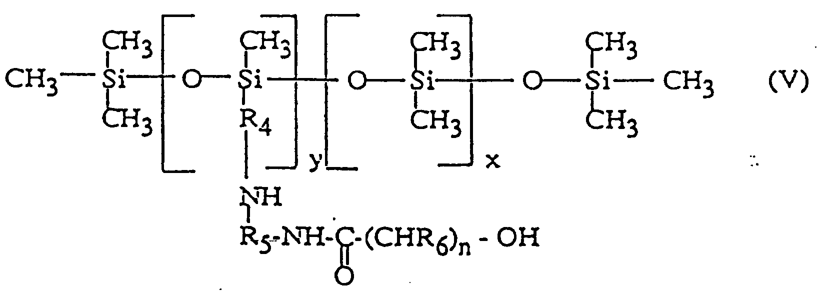 dans laquelle :
R₄ et R₅ désignent (CH₂)₂, (CH₂)₃, (CH₂)₄, -CH₂CH(CH₃)CH₂- ;
R₆ désigne hydrogène, hydroxyle, alkyle, hydroxyalkyle;
n est un nombre entier variant de 2 à 7;
x est un nombre entier variant de 20 à environ 1500;
y varie entre environ 0,5 et 10;
et le rapport y/x est inférieur à 0,05.

7. Composition selon l'une quelconque des revendications 1 à 6, caractérisée par le fait que la silicone est présente dans des proportions comprises entre 0,2 et 30% et de préférence entre 0,4 et 15% en poids par rapport au poids total de la composition.

8. Composition selon l'une quelconque des revendications 1 à 7, caractérisée par le fait qu'elle se présente sous la forme d'un shampooing, de gel douche ou de bain moussant pour le corps.

9. Composition selon l'une quelconque des revendications 1 à 8, caractérisée par le fait qu'elle contient en plus des agents tensio-actifs, différents de ceux de formule (II) telle que définie dans la revendication 1, anioniques, amphotères, zwittérioniques, non ioniques ou leurs mélanges.

10. Composition selon la revendication 9, caractérisée par le fait que les agents tensio-actifs anioniques sont choisis parmi les sels alcalins, les sels de magnésium, les sels d'ammonium, les sels d'amines ou les sels d'amino alcools des composés suivants :
- les alkylsulfates, les alkyléthersulfates, les alkylamidoéthersulfates, les alkylarylpolyéthersulfates, les monoglycéridesulfates;
- les alkylsulfonates, les alkylamidesulfonates, les alkylarylsulfonates, les oléfines sulfonates, les paraffines sulfonates;
- les alkylsulfosuccinates, les alkyléthersulfosuccinates, les alkylamidesulfosuccinates;
- les alkylsulfosuccinamates;
- les alkylsulfoacétates;
- les alkylphosphates, les alkylétherphosphates;
- les acylsarcosinates, les acyliséthionates, les N-acyltaurates, le radical alkyle ou acyle de ces différents composés étant constitué par une chaîne carbonée comportant de 12 à 20 atomes de carbone;
- les sels d'acides gras tels que les sels des acides oléique, ricinoléique, palmitique, stéarique; les acides d'huile de coprah ou d'huile de coprah hydrogénée; les acyl lactylates, dont le radical acyle comporte de 8 à 20 atomes de carbone;
- les acides d'éthers carboxyliques polyoxyalkylénés, répondant à la formule :
R₇-(OC₃H₆)ₚ-(OC₂H₄)ₙ-OCH₂COOA (VI)
dans laquelle R₇ désigne un radical ou un mélange de radicaux alkyle ou alcényle en C₈-C₂₂, linéaire ou ramifié, un radical alkyl(C₈-C₉)phényle, R'₇CONH-CH₂-CH₂ avec R'₇ désignant un radical alkye ou alcényle, linéaire ou ramifié, en C₁₁-C₂₁;
n est un nombre entier ou décimal compris entre 2 et 24,
p est un nombre entier ou décimal compris entre 0 et 6,
A désigne un atome d'hydrogène ou bien Na, K, Li, 1/2 Mg ou un reste monoéthanolamine, ammonium ou triéthanolamine.

11. Composition selon la revendication 9, caractérisée par le fait que le tensio-actif non-ionique est choisi parmi :
a) les alcools, alkylphénols, acides gras polyoxyéthylénés, polypropoxylés ou polyglycérolés à chaîne grasse, comportant 8 à 18 atomes de carbone et comprenant 2 à 50 groupements d'oxyde d'éthylène ou d'oxyde de propylène ou 2 à 30 groupements glycérol;
b) les copolymères d'oxyde d'éthylène et de propylène, les condensats d'oxyde d'éthylène et de propylène sur des alcools gras, les amides gras polyoxyéthylénés, les amides gras polyglycérolés, les amines grasses polyoxyéthylénées, les esters d'acides gras de glycols, les esters d'acides gras du sorbitan oxyéthylénés ou non, les esters d'acides gras de sucrose, les esters d'acides gras de polyéthylèneglycol, les oxydes d'amines.

12. Composition selon la revendication 9, caractérisée par le fait que le tensio-actif amphotère ou zwittérionique est choisi parmi :
a) les dérivés d'amines secondaires ou tertiaires aliphatiques, dans lesquels le radical aliphatique est une chaîne linéaire ou ramifiée de 8 à 18 atomes de carbone et contenant au moins un groupe anionique hydrosoluble carboxylique, sulfonate; sulfate, phosphate ou phosphonate;
b) les alkyl(C₈-C₂₀)bétaïnes, les sulfobétaïnes, les alkyl(C₈-C₂₀) amidoalkyl(C₁-C₆)bétaïnes ou les alkyl(C₈-C₂₀)amidoalkyl(C₁-C₆) sulfobétaïnes.

13. Composition selon l'une quelconque des revendications 9 à 12, caractérisée par le fait que la quantité totale en tensio-actifs autres que les composés de formule (I), est inférieure à 20% en poids par rapport au poids total de la composition.

14. Composition selon l'une quelconque des revendications 1 à 13, caractérisée par le fait que son pH est compris entre 2 et 9.

15. Composition selon l'une quelconque des revendications 1 à 14, caractérisée par le fait qu'elle contient en plus un agent régulateur de viscosité présent dans une concentration pouvant aller jusqu'à 15% en poids et de préférence jusqu'à 6% en poids par rapport au poids total de la composition.

16. Composition selon l'une quelconque des revendications 1 à 15, caractérisée par le fait qu'elle contient en plus des agents de conditionnement des cheveux ou de la peau choisis parmi les agents tensio-actifs cationiques, les polymères, les protéines quaternisées ou non et les silicones hydrosolubles, qui n'altèrent pas la stabilité de la composition, les silicones hydrosolubles étant présentes dans des proportions allant jusqu'à 10% et de préférence comprises entre 0,5 et 6% en poids par rapport au poids total de la composition.

17. Composition selon l'une quelconque des revendications 1 à 16, caractérisée par le fait qu'elle contient en plus, des adjuvants choisis parmi les agents séquestrants, les parfums, les colorants, les conservateurs, les stabilisateurs de mousse, les agents propulseurs, les agents alcalinisants ou acidifiants ou d'autres adjuvants habituellement utilisés en cosmétique.

18. Procédé de lavage et de conditionnement des cheveux ou de la peau, caractérisé par le fait que l'on applique sur ceux-ci une composition telle que définie selon l'une quelconque des revendications 1 à 17, cette application étant suivie d'un rinçage.

19. Composition selon l'une quelconque des revendications 1 à 17, caractérisée par le fait qu'elle contient en plus une substance active pour le traitement des affections dermatologiques.

## Claims

1. Foamable washing composition for hair or skin treatment, characterized in that it contains, in an aqueous medium:
a) a silicone which is insoluble in the said medium and unreactive with the latter, chosen from silicone gums, silicone resins or organomodified silicones containing one or a number of thiol, carboxylate, γ-hydroxypropyl, alkylcarboxylic acid, hydroxyalkylsulphonate, hydroxyalkylthiosulphate or hydroxyacylamino groups, and mixtures thereof, with the exception
(i) of α,ω-trimethyl polydimethylsiloxane gums, and
(ii) of organomodified silicones corresponding to the formula 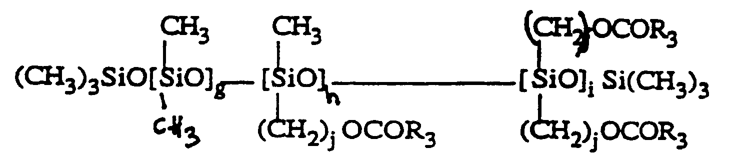 in which g, h and i are individually a number from 1 to 350, j is a number from 0 to 10 and R₃ is an alkyl group having 9 to 21 carbon atoms, and to the formula: 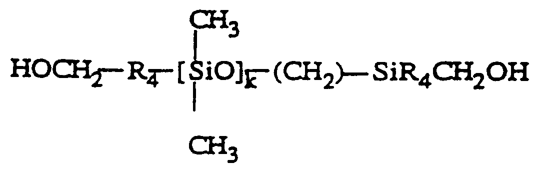 in which k is a number from 1 to 500 and R₄ represents a group Cn₄H₂ₙ₄ where n₄ is a number from 0 to 4;
b) from 4 to 60% of at least one compound corresponding to the following expanded structure: 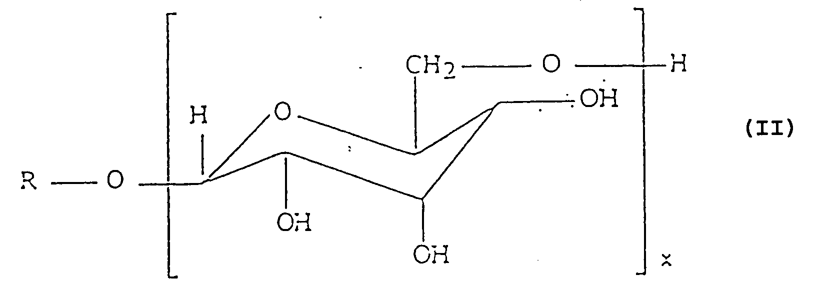 in which:
R denotes a straight- or branched-chain, C₈-C₂₄ alkyl or alkenyl radical or a mixture of straight- or branched-chain, C₈-C₂₄ alkyl or alkenyl radicals;
x is a number from 1 to 15;
the compound of formula (II)/silicone ratio by weight being greater than or equal to 1.

2. Composition according to Claim 1, characterized in that the silicone is chosen from:
a) silicone gums consisting of polydiorganosiloxanes having high molecular weight between 200,000 and 1,000,000; or
b) organopolysiloxane resins which are cross-linked siloxane systems, containing R'₂SiO_{2/2}, R'SiO_{3/2} and SiO_{4/2} units in which R' represents a hydrocarbon group having 1 to 6 carbon atoms or a phenyl group; or else
c) polyalkylsiloxanes, polyarylsiloxanes or polyalkylarylsiloxanes containing, in their structure, one or a number of functional groups as defined in Claim 1, connected to the siloxane chain or connected via a hydrocarbon radical; and mixtures thereof.

3. Composition according to Claim 2, characterized in that the silicone gums are chosen from poly(dimethylsiloxane/methylvinylsiloxane), poly(dimethylsiloxane/diphenylsiloxane), poly(dimethylsiloxane/phenylmethylsiloxane) or poly(dimethylsiloxane/diphenylsiloxane/methylvinylsiloxane) gums.

4. Composition according to Claim 1, characterized in that the organomodified silicones containing γ-hydroxypropyl groups are polyorganosiloxanes corresponding to the formula: 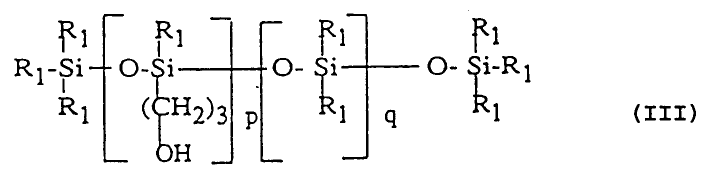 in which:
- the radicals R₁, which are identical or different, are chosen from methyl and phenyl radicals, at least 60 mol % of the radicals R₁ denoting methyl;
- p is between 1 and 30 inclusive;
- q is between 1 and 150 inclusive.

5. Composition according to Claim 1, characterized in that the organomodified silicones containing acyloxyalkyl groups correspond to the formula: 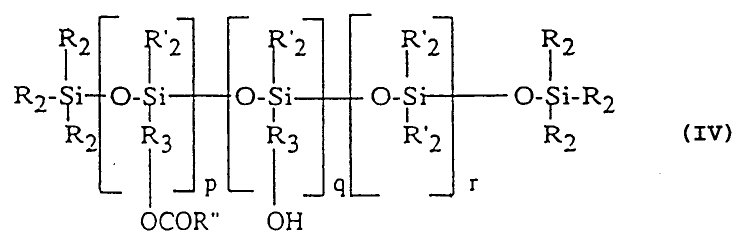 in which
R₂ denotes a methyl, phenyl, -OCOR'' or hydroxyl group, only one of the radicals R₂ per silicon atom may be OH;
R'₂ denotes methyl or phenyl, at least 60 mol % of the radicals R₂ and R'₂ combined denoting methyl;
R'' denotes a C₈-C₂₀ alkyl or alkenyl;
R₃ denotes a linear or branched, divalent C₂-C₁₈ alkylene hydrocarbon radical;
r is between 1 and 120 inclusive;
p is between 1 and 30;
q is equal to 0 or is less than 0.5p, p+q being between 1 and 30; it being possible for the polyorganosiloxanes of formula (IV) to contain 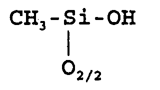 in proportions not exceeding 15% of the sum p+q+r.

6. Composition according to Claim 1, characterized in that the organomodified silicones containing a hydroxyacylamino functional group correspond to the formula: 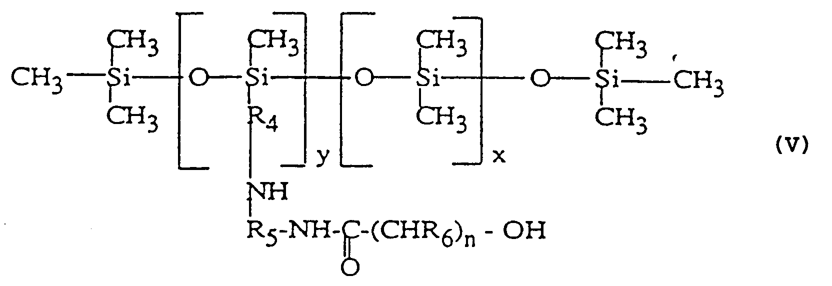 in which:
R₄ and R₅ denote (CH₂)₂, (CH₂)₃, (CH₂)₄ or -CH₂CH(CH₃)CH₂-;
R₆ denotes hydrogen, hydroxyl, alkyl or hydroxyalkyl;
n is an integer varying from 2 to 7;
x is an integer varying from 20 to approximately 1500;
y varies between approximately 0.5 and 10;
and the ratio y/x is less than 0.05.

7. Composition according to any one of Claims 1 to 6, characterized in that the silicone is present in proportions of between 0.2 and 30% and preferably of between 0.4 and 15% by weight with respect to the total weight of the composition.

8. Composition according to any one of Claims 1 to 7, characterized in that it is provided in the form of a shampoo, shower gel or foam bath for the body.

9. Composition according to any one of Claims 1 to 8, characterized in that it additionally contains anionic, amphoteric, zwitterionic or nonionic surface-active agents, or mixtures thereof, different from those of formula (I) as defined in Claim 1.

10. Composition according to Claim 9, characterized in that the anionic surface-active agents are chosen from alkali metal salts, magnesium salts, ammonium salts, amine salts or aminoalcohol salts of the following compounds:
- alkyl sulphates, alkyl ether sulphates, alkylamido ether sulphates, alkyl aryl polyether sulphates or monoglyceride sulphates;
- alkyl sulphonates, alkylamide sulphonates, alkyl aryl sulphonates, olefin sulphonates or paraffin sulphonates;
- alkyl sulphosuccinates, alkyl ether sulphosuccinates or alkylamide sulphosuccinates;
- alkyl sulphosuccinamates;
- alkyl sulphoacetates;
- alkyl phosphates or alkyl ether phosphates;
- acylsarcosinates, acylisethionates or N-acyltaurates,
the alkyl or acyl radical of these various compounds consisting of a carbon chain containing from 12 to 20 carbon atoms;
- fatty acid salts such as the salts of oleic, ricinoleic, palmitic or stearic acids; coconut oil acid or hydrogenated coconut oil acid; or acyllactylates, the acyl radical of which contains from 8 to 20 carbon atoms.
- polyoxyalkylenated ether carboxylic acids, corresponding to the formula:
R₇-(OC₃H₆)ₚ-(OC₂H₄)ₙ-OCH₂COOA (VI)
in which R₇ denotes a linear or branched, C₈-C₂₂ alkyl or alkenyl radical or a mixture of linear or branched, C₈-C₂₂ alkyl or alkenyl radicals a (C₈-C₉)alkylphenyl radical or R'₇CONH-CH₂-CH₂ with R'₇ denoting a linear or branched, C₁₁-C₂₁ alkyl or alkenyl radical;
n is an integer or decimal number between 2 and 24,
p is an integer or decimal number between 0 and 6,
A denotes a hydrogen atom or alternatively Na, K, Li, 1/2 Mg or a monoethanolamine, ammonium or triethanolamine residue.

11. Composition according to Claim 9, characterized in that the nonionic surface-active agent is chosen from:
a) polyoxyethylenated, polypropoxylated or polyglycerolated alcohols, alkylphenols or fatty acids possessing a fatty chain containing 8 to 18 carbon atoms and comprising 2 to 50 ethylene oxide or propylene oxide groups or 2 to 30 glycerol groups;
b) copolymers of ethylene oxide and of propylene oxide, condensates of ethylene oxide and of propylene oxide with fatty alcohols, polyoxyethylenated fatty amides, polyglycerolated fatty amides, polyoxyethylenated fatty amines, fatty acid esters of glycols, oxyethylenated or nonoxyethylenated fatty acid esters of sorbitan, fatty acid esters of sucrose, fatty acid esters of polyethylene glycol or amine oxides.

12. Composition according to Claim 9, characterized in that the amphoteric or zwitterionic surface-active agent is chosen from:
a) derivatives of secondary or tertiary aliphatic amines, in which the aliphatic radical is a linear or branched chain having 8 to 18 carbon atoms, and containing at least one water-soluble carboxyl, sulphonate, sulphate, phosphate or phosphonate anionic group;
b) (C₈-C₂₀)alkyl betaines, sulphobetaines, (C₈-C₂₀)alkylamido(C₁-C₆)alkyl betaines or (C₈-C₂₀)alkylamido(C₁-C₆)alkyl sulphobetaines.

13. Composition according to any one of Claims 9 to 12, characterized in that the total amount of surface-active agents other than the compounds of formula (I) is less than 20% by weight with respect to the total weight of the composition.

14. Composition according to any one of Claims 1 to 13, characterized in that its pH is between 2 and 9.

15. Composition according to any one of Claims 1 to 14, characterized in that it additionally contains a viscosity regulating agent present in a concentration which can range up to 15% by weight, and preferably up to 6% by weight, with respect to the total weight of the composition.

16. Composition according to any one of Claims 1 to 15, characterized in that it additionally contains hair or skin conditioning agents chosen from cationic surface-active agents, polymers, quaternized or nonquaternized proteins and water-soluble silicones, which do not detrimentally affect the stability of the composition, the water-soluble silicones being present in proportions ranging up to 10%, and preferably between 0.5 and 6%, by weight with respect to the total weight of the composition.

17. Composition according to any one of Claims 1 to 16, characterized in that it additionally contains adjuvants chosen from sequestering agents, fragrances, dyes, preserving agents, foam stabilizing agents, propellants, basifying or acidifying agents or other adjuvants commonly used in cosmetics.

18. Process for washing and conditioning hair or the skin, characterized in that a composition as defined according to any one of Claims 1 to 17 is applied to hair or the skin, this application being followed by rinsing.

19. Composition according to any one of Claims 1 to 17, characterized in that it additionally contains an active substance for treatment of dermatological ailments.

## Patentansprüche

1. Schäumende Waschzusammensetzung zur Behandlung der Haare oder der Haut,
dadurch **gekennzeichnet**, daß
sie in einem wässrigen Milieu enthält:
a) ein Silikon, das in dem genannten Milieu unlöslich und nicht damit reaktiv ist, ausgewählt aus gummiartigen Produkten aus Silikon, Harzen aus Silikon, aus Silikonen, die mit einer oder mehreren Thiol-, Carboxylat-, γ-Hydroxypropyl-, Alkylcarboxylsäure-, Hydroxyalkylsulfonat-, Hydroxyalkylthiosulfat- oder Hydroxyacylamino-Gruppierungen organomodifiziert sind, sowie aus deren Mischungen, ausgenommen
(i) Gummi-Produkte aus α,ω*'*-Trimethylpolydimethylsiloxan und
(ii) organomodifizierte Silikone der Formel
(CH₃)SiO[Si(CH₃)₂O]_{g}-[Si(CH₃)((CH₂)ⱼOCOR₃)O]ₕ- [Si((CH₂)ⱼOCOR₃)₂O]Si(CH₃)₃
worin g, h und i jeweils eine Zahl von 1 bis 350,
j eine Zahl von 0 bis 10 und R₃ ewine Alkylgruppe mit 9 bis 21 Kohlenstoffatomen sind,
sowie die Formel:
HOCH₂-R₄-[Si(CH₃)₂O]ₖ-(CH₂)-SiR₄CH₂OH
worin k eine Zahl von 1 bis 500 ist und R₄ eine Cₙ₄H₂ₙ₄ -Gruppe darstellt, worin n4 eine Zahl von 0 bis 4 ist;
b) 4 bis 60 Prozent mindestens einer Verbindung der dargestellten Struktur (II); 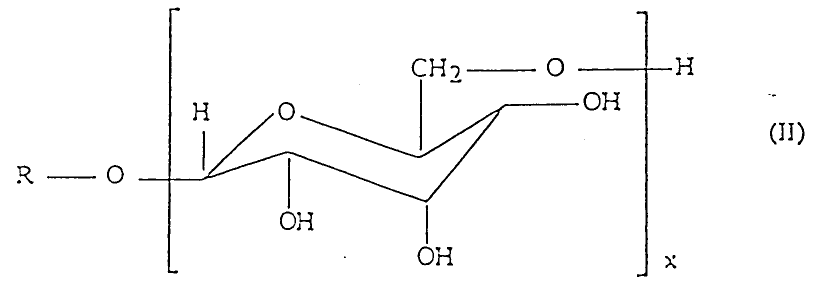 worin gilt:
R bezeichnet einen geradkettigen oder verzweigten C₈₋₂₄-Alkyl- oder -Alkenylrest oder eine Mischung aus diesen Resten, und
x ist eine Zahl von 1 bis 15,
worin das Gewichtsverhältnis: Verbindung der Formel (II)/unlösliches Silikon mehr als oder gleich 1 beträgt.

2. Zusammensetzung gemäß Anspruch 1,
dadurch **gekennzeichnet**, daß
das Silikon ausgewählt ist aus:
a) gummiartigen Produkten von Silikonen, aufgebaut aus Polydiorganosiloxanen mit erhöhtem Molekulargewicht von 200000 bis 1000000; oder
b) Harzen von Organopolysiloxanen, die vernetzte Siloxan-Systeme sind, umfassend Einheiten R'₂SiO_{2/2}, R'SiO_{3/2} und SiO_{4/2}, in denen R' eine Kohlenwasserstoffgruppe mit 1 bis 6 Kohlenstoffatomen oder eine Phenylgruppe darstellt; oder auch
c) aus Polyalkylsiloxanen, Polyarylsiloxanen oder Polyalkylarylsiloxanen, welche in ihrer Struktur eine oder mehrere in Anspruch 1 definierte funktionelle Gruppierungen aufweisen, die an die Siloxankette direkt oder über das Zwischenglied eines Kohlenwasserstoffrestes gebunden sind, sowie aus deren Mischungen.

3. Zusammensetzung gemäß Anspruch 2,
dadurch **gekennzeichnet**, daß
die gummiartigen Silikon-Produkte ausgewählt sind aus gummiartigen Produkten von
Poly(dimethylsiloxan/methylvinylsiloxan),
Poly(dimethylsiloxan/diphenylsiloxan),
Poly(dimethylsiloxan/phenylmethylsiloxan),
Poly(dimethylsiloxan/diphenylsiloxan/methylvinylsiloxan).

4. Zusammensetzung gemäß Anspruch 1,
dadurch **gekennzeichnet**, daß
die mit γ-Hydroxypropylgruppen organomodifizierten Silikone Polyorganosiloxane der Formel sind: 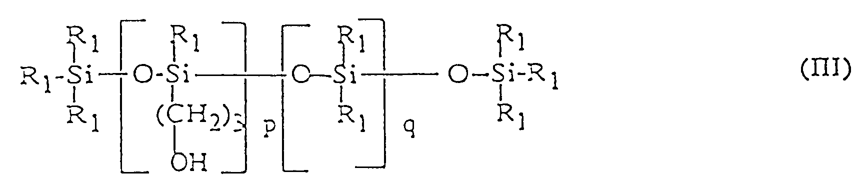 worin gilt:
- die Reste R₁ sind, gleich oder verschieden, aus Methyl- und Phenylresten ausgewählt, wobei mindestens 60 Mol% der Reste R₁ durch den Methylrest dargestellt sind;
- p beträgt 1 bis 30;
- q beträgt 1 bis 150.

5. Zusammensetzung gemäß Anspruch 1,
dadurch **gekennzeichnet**, daß
die mit Acyloxyalkylgruppen organomodifizierten Silikone die Formel aufweisen: 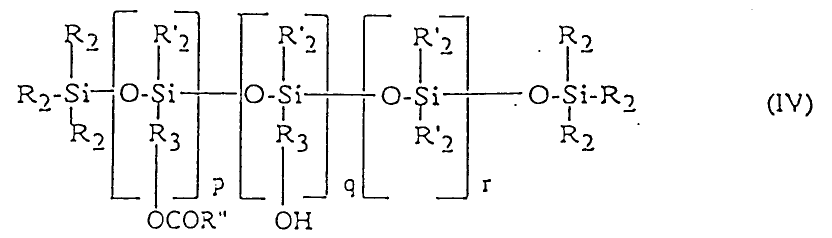 worin gilt:
R₂ bedeutet eine Methyl-, Phenyl-, -OCOR''- oder eine Hydroxylgruppe, wobei einer der Reste R₂ pro Silizium OH sein kann;
R'₂ bedeutet einen Methyl- oder Phenylrest, wobei mindestens 60 Mol% aller Reste R₂ und R'₂ den Methylrest darstellen;
R'' bedeutet einen C₈₋₂₀-Alkyl- oder -Alkenylrest;
R₃ bedeutet einen linearen oder verzweigten zweiwertigen C₂₋₁₈-Alkylenkohlenwasserstoffrest;
r beträgt 1 bis 120;
p beträgt 1 bis 30;
q = 0 oder weniger als 0,5 p, wobei p + q 1 bis 30 beträgt, und wobei die Polyorganosiloxane der Formel (IV) CH₃-Si-O_{2/2}-OH-Gruppierungen in Mengenanteilen enthalten können, die 15% der Summe p+q+r nicht übersteigen.

6. Zusammensetzung gemäß Anspruch 1,
dadurch **gekennzeichnet**, daß
die mit Hydroxyacylamino-Funktion organomodifizierten Silikone die Formel aufweisen: 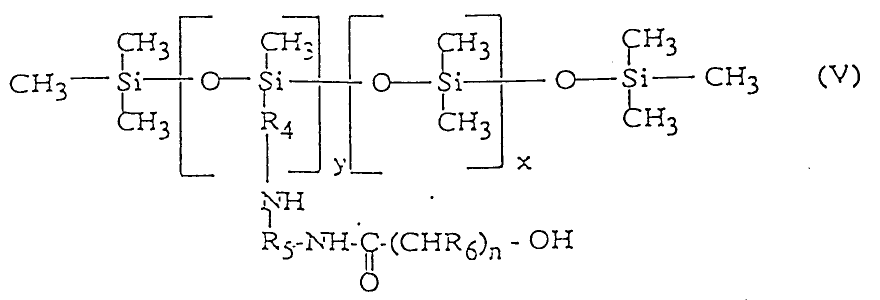 worin gilt:
R₄ und R₅ bedeuten (CH₂)₂, (CH₂)₃, (CH₂)₄, -CH₂CH(CH₃)CH₂-,
R₆ bedeutet Wasserstoff, Hydroxyl, Alkyl, Hydroxyalkyl,
n ist eine ganze Zahl von 2 bis 7,
x ist eine ganze Zahl von 20 bis ca. 1500,
y beträgt ca. 0,5 bis 10,
wobei das Verhältnis y/x weniger als 0,05 beträgt.

7. Zusammensetzung gemäß jedem der Ansprüche 1 bis 6,
dadurch **gekennzeichnet**, daß
das Silikon in Mengenanteilen von 0,2 bis 30 und vorzugsweise von 0,4 bis 15 Gew.%, bezogen auf das Gesamtgewicht der Zusammenseztung, vorhanden ist.

8. Zusammensetzung gemäß jedem der Ansprüche 1 bis 7,
dadurch **gekennzeichnet**, daß
sie in Form eines Shampoo, Duschgels oder Badeschaums für den Körper vorliegt.

9. Zusammensetzung gemäß jedem der Ansprüche 1 bis 8,
dadurch **gekennzeichnet**, daß
sie zusätzlich anionische, amphotere, zwitterionische oder nichtionische oberflächenaktive Mittel oder deren Mischungen enthält, welche von den in Anspruch 1 definierten Mitteln der Formel (II) verschieden sind.

10. Zusammensetzung gemäß Anspruch 9,
dadurch **gekennzeichnet**, daß
die anionischen oberflächenaktiven Mittel aus Alkali-, Magnesium-, Ammonium-, Amin- oder Alkoholamino-Salzen der folgenden Verbindungen, und zwar von:
- Alkylsulfaten, Alkylethersulfaten, Alkylamidoethersulfaten, Alkylarylpolyethersulfaten, Monoglyceridsulfaten,
- Alkylsulfonaten, Alkylamidsulfonaten, Alkylarylsulfonaten, Olefinsulfonaten, Paraffinsulfonaten,
- Alkylsulfosuccinaten, Alkylethersulfosuccinaten, Alkylamidsulfosuccinaten,
- Alkylsulfosuccinamaten,
- Alkylsulfoacetaten,
- Alkylphosphaten, Alkyletherphosphaten,
- Acylsarcosinaten, Acylisethionaten, N-Acyltauraten,
wobei der Alkyl- oder Acylrest dieser verschiedenen Verbindungen im allgemeinen aus einer Kohlenwasserstoffkette mit 12 bis 20 Kohlenstoffatomen aufgebaut ist;
- aus Fettsäuresalzen wie den Salzen von Öl-, Rizinolein-, Palmitin- oder Stearinsäure; aus Säuren vom Öl von Kopra oder vom Öl von hydriertem Kopra; aus Acyllactylaten, deren Acylrest 8 bis 20 Kohlenstoffatome aufweist;
- aus polyoxalkylierten Carboxylsäureethern der Formel:
R₇-(OC₃H₆)ₚ-(OC₂H₄)ₙ-OCH₂COOA (VI)
worin R₇ einen linearen oder verzweigten C₈₋₂₂-Alkyl- oder -Alkenyl-Rest oder eine Mischung dieser Reste, einen C₈₋₉-Alkylphenyl-Rest, R'₇CONH-CH₂-CH₂ bedeutet, worin R'₇ einen linearen oder verzweigten C₁₁₋₂₁-Alkyl- oder -Alkenyl-Rest darstellt,
n eine Zahl oder Dezimalzahl von 2 bis 24 ist,
p eine Zahl oder Dezimalzahl von 0 bis 6 ist und
A ein Wasserstoffatom oder auch Na, K. Li, ½ Mg oder einen Monoethanolamin-, Ammonium- oder Triethanolamin-Rest bedeutet,
ausgewählt sind.

11. Zusammensetzung gemäß Anpsruch 9,
dadurch **gekennzeichnet**, daß das nicht-ionische oberflächenaktive Mittel ausgewählt ist aus:
a) Alkoholen, Alkylphenolen und polyoxethylierten, polypropoxylierten oder polyglycerierten Fettsäuren mit einer Fettkette von 8 bis 18 Kohlenstoffatomen, wobei die Zahl der Ethylen- oder Propylenoxid-Gruppierungen 2 bis 50 und die Zahl der Glyceryl-Gruppierungen 2 bis 30 betragen;
b) Copolymeren von Ethylen- und Propylenoxid, Kondensaten aus Ethylen- und Propylenoxid an Fettalkohole, polyoxethylierten Fettamiden, polyglycerierten Fettamiden, polyoxethylierten Fettaminen, Fettsäureestern von Glycolen, oxethylierten oder nicht-oxethylierten Fettsäuresorbitanestern, Fettsäureestern von Sucrose, Fettsäureestern von Polyethylenglycol oder aus Aminoxiden.

12. Zusammensetzung gemäß Anspruch 9,
dadurch **gekennzeichnet,** daß
das amphotere oder zwitterionische oberflächenaktive Mittel ausgewählt ist aus:
a) Derivaten von sekundären oder tertiären aliphatischen Aminen, in denen der aliphatische Rest eine lineare oder verzweigte, 8 bis 18 Kohlenstoffatome aufweisende Kette ist, die mindestens eine anionische wasserlösliche Carboxy-, Sulfonat-, Sulfat-, Phosphat oder Phosphonat-Gruppe enthält,
b) C₈₋₂₀-Alkylbetainen, Sulfobetainen, C₈₋₂₀-Alkylamido-C₁₋₆-alkylbetainen oder C₈₋₂₀-Alkylamido-C₁₋₆-alkylsulfobetainen.

13. Zusammensetzung gemäß jedem der Ansprüche 9 bis 12,
dadurch **gekennzeichnet**, daß
die Gesamtmenge der von den Verbindungen der Formel (II) verschiedenen oberflächenaktiven Mittel weniger als 20 Gew.%, bezogen auf das Gesamtgewicht der Zusammensetzung, beträgt.

14. Zusammensetzung gemäß jedem der Ansprüche 1 bis 13,
dadurch **gekennzeichnet**, daß
ihr pH-Wert 2 bis 9 beträgt.

15. Zusammensetzung gemäß jedem der Ansprüche 1 bis 14,
dadurch **gekennzeichnet**, daß
sie zusätzlich ein Reguliermittel der Viskosität in einer Konzentration bis zu 15 und vorzugsweise bis zu 6 Gew.%, bezogen auf das Gesamtgewicht der Zusammensetzung, enthält.

16. Zusammensetzung gemäß jedem der Ansprüche 1 bis 15,
dadurch **gekennzeichnet**, daß
sie zusätzlich Mittel zur Konditionierung der Haare oder der Haut enthält, die aus kationischen oberflächenaktiven Mitteln, Polymeren, Proteinen, die quaterniert sind oder nicht, und aus wasserlöslichen Silikonen ausgewählt sind, wobei diese Mittel die Stabilität der Zusammensetzung nicht verändern, und wobei die wasserlöslichen Silikone in Mengenanteilen bis zu 10 und vorzugsweise von 0,5 bis 6 Gew.%, bezogen auf das Gesamtgewicht der Zusammensetzung, vorhanden sind.

17. Zusammensetzung gemäß jedem der Ansprüche 1 bis 16,
dadurch **gekennzeichnet**, daß
sie zusätzlich Hilfsstoffe aus Sequestriermitteln, Parfüm-Produkten, Färbemitteln, Konservierungsstoffen, Schaumstabilisatoren, Treibmitteln, alkalisch oder sauer stellenden Mitteln oder aus weiteren, gewöhnlich in der Kosmetik eingesetzten Hilfsstoffen enthält.

18. Verfahren zum Waschen und Konditionieren der Haare oder der Haut, dadurch **gekennzeichnet**, daß
man auf diese eine in jedem der Ansprüche 1 bis 17 definierte Zusammensetzung aufbringt, worauf eine Spülung erfolgt.

19. Zusammensetzung gemäß jedem der Ansprüche 1 bis 17,
dadurch **gekennzeichnet**, daß
sie zusätzlich eine Wirkstoffsubstanz zur Behandlung dermatologischer Affektionen enthält.
